# EUROPÄISCHE PATENTSCHRIFT

(11) **EP 0 794 758 B1**
(45) Veröffentlichungstag und Bekanntmachung des Hinweises auf die Patenterteilung: **12.05.1999**
(21) Anmeldenummer: 96900913.3
(22) Anmeldetag: 08.01.1996
(51) Int. Cl.: A61K 7/00

(54) **2-PHASEN-HAARBEHANDLUNGSMITTEL**
2-PHASE HAIR TREATMENT MEDIUM III
AGENT DE SOINS CAPILLAIRES A DEUX PHASES

(30) Priorität: 17.01.1995 DE 19501188
(43) Veröffentlichungstag der Anmeldung: 17.09.1997
(73) Patentinhaber: Henkel Kommanditgesellschaft auf Aktien, 40191 Düsseldorf (DE)
(72) Erfinder: HOLLENBERG, Detlef, 40699 Erkrath (DE); SEIDEL, Kurt, 40589 Düsseldorf (DE); PRIEBE, Christian, 42489 Wülfrath (DE)
(86) Internationale Anmeldenummer: EP9600040
(87) Internationale Veröffentlichungsnummer: WO9622070

(56) Entgegenhaltungen:
- DE-C- 4 100 490
- DE-C- 4 426 952

## Beschreibung

Die Erfindung betrifft Mittel, insbesondere kosmetische Mittel, in Form von 2-Phasen-Zubereitungen.

Das menschliche Haupthaar wird heute in vielfältiger Weise mit haarkosmetischen Zubereitungen behandelt. Dazu gehört beispielsweise die Reinigung der Haare mit Shampoos und Duschpräparaten und das Bleichen, Färben und Verformen von Haaren mit Wellmitteln, Tönungsmitteln und Stylingpräparaten. Als Folge von Haarbehandlungen kann es zu unerwünschten Beeinträchtigungen der Haarstruktur kommen. Diese Beeinträchtigungen Zeigen sich u.a. in einer schlechten Naß- und Trockenkämmbarkeit, einer verstärkten elektrostatischen Aufladung, verstärkter Sprödigkeit sowie in gravierenderen Fällen in gesplißten, d.h. aufgespaltenen Haar(end)en. Dies verschlechtert nicht nur das äußere Erscheinungsbild der Frisur, sondern insbesondere dem Haarspliß sollte auch aufgrund der Haargesundheit entgegengewirkt werden.

Es wurde daher eine Reihe von Produkten entwickelt, die eine oder mehrere der folgenden Aufgaben erfüllen:
- Abbau der durch eine Haarbehandlung verursachten Schädigungen durch Regeneration des Haares,
- Vermeidung oder Entgegenwirkung der durch Haarschädigungen hervorgerufenen negativen Auswirkungen auf das äußere Erscheinungsbild der Frisur und die Handhabbarkeit der Haare sowie
- Prävention möglicher, durch Umwelteinflüsse oder Haarbehandlungen hervorgerufener, Haarschädigungen durch gezielte Zufuhr von Wirkstoffen.

Eine Reihe solcher Produkte sind unter Bezeichnungen wie "Haarnachbehandlungsmittel", "Pflegespülung", "Haarwasser", "Haarkur", "Haarbalsam" etc. auf dem Markt. Diese Mittel werden in der überwiegenden Zahl der Fälle als Emulsionen oder wäßrige Lösungen formuliert. Diese Formulierungen erlauben eine gleichmäßige Verteilung der Wirkstoffe auf dem Substrat; das Gefühl nach dem Auftragen wird vom Verbraucher in den allermeisten Fällen als angenehm empfunden.

Eine solche Darreichungsform ist aber nur dann möglich, wenn es die zu verwendenden Wirkstoffe erlauben, daß heißt die Verträglichkeiten von Wirk-, Zusatz- und Hilfsstoffen hinreichend hoch ist.

Weiterhin gibt es ein ständig wachsendes Bemühen, aus ökologischen auf Teile des Verpackungsmaterials zu verzichten. Wurde früher der Verbraucher häufig durch eine originelle Verpackungsform angesprochen, so wird heute z.B. immer mehr auf die für diesen Zwecke geeigneten Umkartons verzichtet. Ein Weg, das Interesse des Verbrauchers für das Produkt zu wecken, besteht darin, dieses so effektvoll und ansprechend zu formulieren, daß es auch in einer einfachen Glasflasche die Aufmerksamkeit des Konsumenten erregt. Dieses Ziel kann beispielsweise durch mehrphasige Zubereitungen erreicht werden, die auch aus anderen Gründen (Wirkstoffinkompatibilitäten) eine bevorzugte Formulierungsform darstellen können.

Mitentscheidend für den Erfolg des Bemühens, mittels einer z.B. 2-phasigen Zubereitung das Interesse des Verbrauchers zu wecken, ist die farbliche Gestaltung des Produktes. So werden die Phasen durch mindestens 2 unterschiedliche Farbstoffe, von denen jeweils einer in jeder der beiden Phasen löslich ist, auch farblich differenziert. Hierbei kommt der Auswahl der Farbstoffe aus vielerlei Gründen große Bedeutung bei. Es seien hier nur das Harmonieren der Farben aus Sicht des Verbrauchers, die toxikologische und dermatologische Verträglichkeit der Farbstoffkomponenten sowie deren Verträglichkeit mit den übrigen Komponenten der Zubereitung genannt. Eine condition sine qua non ist aber eine hinreichende Stabilität der Farbstoffe gegenüber dem Tageslicht, um dem Verbraucher ein immer gleich ansprechend aussehendes Produkt zu garantieren.

Es wurde nun gefunden, daß auch ein gegen Tageslicht weniger stabiler Inhaltsstoff sich als Komponente von dem Tageslicht ausgesetzten 2-Phasen-Produkten in hervorragender Weise eignen, wenn neben diesem Inhaltsstoff in der gleichen Phase eine Substanz mit UV-Schutzwirkung gelöst ist.

Gegenstand der Erfindung ist somit eine in Form eines 2-Phasen-Systems vorliegende, durch mechanische Einwirkung kurzzeitig mischbare Zubereitung zur Behandlung keratinischer Gebilde, insbesondere menschlicher Haare und menschlicher Haut, dadurch gekennzeichnet, daß sie mindestens einen gelösten, gegen Tageslicht instabilen Inhaltsstoff und, in der gleichen Phase gelöst, einen UV-Filter, enthält.

Das Prinzip kurzzeitig mischbarer 2-Phasen-Formulierungen ist beispielsweise aus den Druckschriften DE-OS 16 17 808, DE-C1-36 27 313, DE-A1-29 05 257, DE-A1-31 10 258, DE-C1-4100490 und DE-C1 42 41 799 bekannt. In Mitteln gemäß dieser Druckschriften werden 2-Phasen-Zubereitungen insbesondere deshalb verwendet, um öllösliche Wirkstoffe in Form einer, vom Verbraucher gefühlsmäßig bevorzugten, Emulsion auftragen zu können. Die Herstellung in Form einer stabilen Emulsion ist aber nicht oder nur unter Schwierigkeiten möglich, auf entsprechende Emulgatore(menge)n oder Stabilisierungsmittel soll aus anderen Erwägungen verzichtet werden oder die Emulsionen haben sich bei der Anwendung auf Haut oder Haar als weniger geeignet erwiesen.

Im Rahmen der vorliegenden Erfindung ist der Begriff "kurzzeitig mischbar" durch folgenden Test definiert:
Die 2-Phasen-Zubereitung wird in ein Reagenzglas mit ca. 1 cm Durchmesser in einer solchen Menge gebracht, daß mindestens 90 % des Volumens gefüllt sind. Das verschlossene Reagenzglas wird jeweils durch Drehen um die kurze Achse um 180° 5 mal innerhalb von 5 Sekunden "auf den Kopf gestellt" und wieder in die Ausgangslage gebracht. Dann wurde das Reagenzglas ruhig in der ursprünglichen Lage belassen. Eine 2-Phasen-Zubereitung gilt als "kurzfristig mischbar" im Sinne der Erfindung, wenn bei Betrachtung mit dem bloßen Auge folgende 3 Bedingungen erfüllt waren:
1. Durch die Bewegung bildet sich ein 1-Phasen-System,
2. 5 - 30 Sekunden nach Ende der Bewegung ist das Auftreten einer zweiten Phase erkennbar,
3. nach spätestens 30 Stunden ist die Phasentrennung vollständig abgeschlossen.

Dabei haben sich solche Systeme als anwendungstechnisch besonders geeignet erwiesen, bei denen die Abtrennung der 2. Phase (Bedingung 2) nach 10-20 Sekunden begann und nach 24 Stunden beendet ist (Bedingung 3).

Bei den beiden Phasen kann es sich um eine rein wäßrige Phase, gegebenenfalls mit weiteren gelösten Bestandteilen, und um eine reine Öl-Phase, ebenfalls gegebenenfalls mit weiteren gelösten Bestandteilen, handeln.

Üblicherweise wird aber die Wasserphase Ölanteile und die Ölphase Wasseranteile aufweisen.

Dabei ist bevorzugt, daß die wäßrige Phase entweder keine, oder nur geringe Ölanteile enthält. Eine solche Emulsion hat dann bevorzugt nicht mehr als 5 Gew.-% Ölanteile, bezogen auf die Komponenten Wasser und Öl.

Als Öl-Phase wird in der Regel eine Emulsion vorliegen, die bis zu 20 Gew.-% Wasser, bezogen auf die Komponenten Öl und Wasser, enthält.

Die erfindungsgemäßen Zubereitungen enthalten die beiden Phasen bevorzugt in einem Verhältnis von 1:1 bis 5:1. Massenverhältnisse von 2:1 bis 1:2 von Öl-Phase und Wasserphase sind dabei besonders bevorzugt.

Öle im Sinne der Erfindung sind sowohl mineralische Öle, pflanzliche Öle und synthetische Öle, wie beispielsweise Silikonöle. Wegen ihrer besonderen anwendungstechnischen Eigenschaften kann es bevorzugt sein, als Öl-phase Paraffine oder Silikonöle, insbesondere Polydimethylsiloxane niedriger Viskositäten, einzusetzen. Ein entsprechendes Silikonöl ist beispielsweise unter der Bezeichnung Dow Corning^{R}200 Fluid im Handel.

Instabil im Sinne der Erfindung sind Inhaltsstoffe, wenn sie sich nicht zur Formulierung üblicher, über den Einzelhandel abgegebener Produkte eignen. Im Falle von Farbstoffen zur Anfärbung der Produkte ist dies der Fall, wenn eine durch übliche Mengen dieser Farbstoffe angefärbte flüssige Phase bei Lagerung bei Raumtemperatur (20 °C) vor Ablauf von 2 Jahren mit dem bloßen Auge erkennbare Farbveränderungen aufweisen. Im Falle von Wirkstoffen ist dies der Fall, wenn im gleichen Zeitraum mehr als die Hälfte der entsprechenden Verbindungen abgebaut sind. Dem Fachmann sind die Lichtechtheiten üblicher kosmetischer Inhaltsstoffe bekannt; sie können häufig auch den Datenblättern, die von den jeweiligen Herstellern erhältlich sind, entnommen werden. Weiterhin sind dem Fachmann eine Reihe von Tests unter verschärften Bedingungen bekannt, mit denen er sich relativ schnell über die Lichtechtheit von Substanzen informieren kann. Ein Beispiel für einen solchen Test ist die Lichtechtheitsbestimmung nach DIN 54004. Für Farbstoffe liegt die zur Anfärbung von erfindungsgemäßen Kosmetika notwendige Lichtechtheit der entsprechenden Farbstoffe in der Regel bei Typ 3. Dieser Lichtechtheitstest läßt sich auch zur Bestimmung von Wirkstoffstabilitäten in Lösung in für den Fachmann naheliegender Weise abwandeln.

Eine Gruppe erfindungsgemäß bevorzugter, lichtempfindlicher Inhaltsstoffe stellen wasserlösliche Farbstoffe dar. Beispiele für solche, insbesondere zur Anfärbung kosmetischer Produkte geeigneter Farbstoffe stellen die im Handel unter den Bezeichnungen D&C Yellow No.10 (C.I. 47005), D&C Yellow No.6 (C.I. 15985), D&C Green No.3 (C.I. 42053) und Sicomet Blau (C.I. 74160) dar.

Eine weitere Gruppe erfindungsgemäß bevorzugter, lichtempfindlicher Inhaltsstoffe stellen öllösliche Farbstoffe dar. Beispiele für solche, insbesondere zur Anfärbung kosmetischer Produkte geeigneter Farbstoffe stellen die im Handel unter den Bezeichnungen D&C Violet No.2 (C.I. 60725), D&C Green No.6 (C.I. 61565), D&C Red No.17 (C.I. 26100) und D&C Yellow No. 11 erhältlichen Produkte sowie die Naturprodukte Chlorophyll und β-Carotin dar.

Weitere Gruppen erfindungsgemäß bevorzugter, lichtempfindlicher Inhaltsstoffe stellen wasserlösliche Vitamine und Antischuppenwirkstoffe dar. Beispiele für diese Gruppen sind Vitamin B₁₂ sowie der unter der CTFA-Bezeichnung Piroctone Olamine bekannte Antischuppenwirkstoff.

Eine wichtige Gruppe lichtempfindlicher, öllöslicher Inhaltsstoffe stellen schließlich Vitamine dar. Als Beispiele seien schließlich die Vitamine A, D₂ und E genannt.

Entsprechend den zu schützenden Inhaltsstoffen müssen als UV-Filter öl- oder wasserlösliche Verbindungen verwendet werden. Sind sowohl öl- als auch wasserlösliche Inhaltsstoffe zu schützen, so wird man in der Regel eine Kombination aus mindestens einem wasserlöslichen und mindestens einem öllöslichen UV-Filter einsetzen.

Im Handel ist sowohl eine Palette öllöslicher als auch wasserlöslicher UV-Filter in für kosmetische Zwecke geeigneten Reinheiten erhältlich. Die meisten dieser Verbindungen basieren auf wenigen Grundgerüsten, deren Eigenschaften wie Absorptionsvermögen und Löslichkeitsverhalten durch entsprechende Substituenten die gewünschten Werte aufweisen. Solche Grundgerüste sind beispielsweise Benzophenon, p-Aminobenzoesäureester, Diphenylacrylsäureester, Zimtsäureester, Salicylsäureester, Benzimidazole und o-Aminobenzoesäureester. UV-Filter auf Basis von Benzophenonen und p-Aminobenzoesäureestern können erfindungsgemäß besonders bevorzugt sein.

Beispiele für erfindungsgemäß bevorzugte öllösliche Farbstoffe sind die unter den CTFA-Bezeichnungen bekannten Verbindungen Benzophenone-3, Benzophenone-2, Benzophenone-6, Benzophenone-1, Benzophenone-11, Etocrylene, Octocrylene, Octyl Methoxycinnamate, Octyl Salicylate und Menthyl Anthranile.

Beispiele für erfindungsgemäß bevorzugte wasserlösliche Farbstoffe sind die unter den CTFA-Bezeichnungen bekannten Verbindungen Benzophenone-4, Benzophenone-9, PEG-25 PABA und Phenylbenzimidazole Sulfonic Acid.

Um die Zubereitungen mit den erfindungsgemäßen Eigenschaften herzustellen, werden diese in der Regel mindestens einen Emulgator enthalten.

Als besonders geeignet für die Herstellung solcher Systeme haben sich beispielsweise kationische derivatisierte Panthenole erwiesen. Solche Verbindungen sind beispielsweise aus der PCT-Offenlegungsschrift WO 92/13829 bekannt, auf die hier ausdrücklich Bezug genommen wird. Ein entsprechendes Produkt wird von der Firma Tri-K unter der Bezeichnung PANTHEQUAT^{R} vertrieben.

Ebenfalls hervorragend geeignet für die Herstellung solcher Systeme sind bestimmte Polyoxyalkylen-Ether von Polyglycerin-Fettsäureestern. Diese Verbindungen enthalten üblicherweise
- 2 bis 20 Oxyalkylen-, insbesondere Oxyethylen-, Einheiten,
- 2 bis 10 Glycerin-Einheiten sowie
- 1 oder 2 Fettsäureeinheiten mit 8 bis 24 Kohlenstoffatomen.

Verbindungen mit bis zu 5 Glycerin-Einheiten, Oxyethylen-Einheiten und einer Fettsäureeinheit haben sich als erfindungsgemäß besonders geeignet erwiesen. Die Fettsäureeinheiten können sowohl gesättigt als auch ungesättigt sein; Verbindungen auf Basis Laurin-, Myristin-, Palmitin-, Stearin- und Öl-Säure haben sich als besonders geeignet erwiesen.

Emulgatoren dieses Typs sind ebenfalls im Handel erhältlich; so vertreibt die Hoechst AG unter der Bezeichnung Hostacerin^{R}-DGL einen Poly(10)ethylenglykolether eines mit Laurinsäure veresterten Diglycerins.

Diese Emulgatoren sind in den erfindungsgemäßen Zubereitungen bevorzugt in einer Menge von 0,05 bis 1 Gew.-%, insbesondere 0,05 bis 0,5 Gew-%, bezogen auf die gesamte Zubereitung, enthalten.

Entsprechend der Aufgabe der erfindungsgemäßen Zubereitung enthält diese in der Regel noch weitere kosmetische Wirkstoffe, sowie übliche Hilfs- und Zusatzstoffe. In den meisten Fällen unterliegen die erfindungsgemäßen Zubereitungen hinsichtlich dieser Bestandteile keinen Beschränkungen. Lediglich bei weiteren Tensiden, die das Mischungsverhalten der beiden Phasen und die Emulsionsstabilität stark beeinflussen können, muß der Fachmann die Eignung mit Hilfe des oben genannten einfachen Testes gegebenenfalls überprüfen; gleiches gilt für den Fall, daß große Mengen an viskositätssteigernden Mitteln eingesetzt werden sollen.

Übliche Wirk-, Hilfs- und Zusatzstoffe für die erfindungsgemäßen Zubereitungen sind beispielsweise
- anionische Tenside, wie beispielsweise Fettalkylsulfate- und -ethersulfate sowie Alkylethercarbonsäuren,
- zwitterionische Tenside, wie beispielsweise Betaine,
- ampholytische Tenside,
- nichtionogene Tenside, wie beispielsweise Alkylpolyglycoside und ethoxylierte Fettalkohole,
- kationische Tenside, wie beispielsweise quartäre Ammoniumverbindungen, Amidoamine und sogenannte "Esterquats"
- nichtionische Polymere wie beispielsweise Vinylpyrrolidon/Vinylacrylat-Copolymere, Polyvinylpyrrolidon und Vinylpyrrolidon/Vinylacetat-Copolymere und Polysiloxane,
- kationische Polymere wie quaternisierte Celluloseether, Polysiloxane mit quaternären Gruppen, Dimethyldiallylammoniumchlorid-Polymere, Acrylamid-Dimethyldiallylammoniumchlorid-Copolymere, mit Diethylsulfat quaternierte Dimethylaminoethylmethacrylat-Vinylpyrrolidon-Copolymere, Vinylpyrrolidon-Imidazoliniummethochlorid-Copolymere und quaternierter Polyvinylalkohol,
- zwitterionische und amphotere Polymere wie beispielsweise Acrylamidopropyl-trimethylammoniumchlorid/Acrylat-Copolymere und Octylacrylamid/Methyl-methacrylat/tert.Butylaminoethylmethacrylat/2-Hydroxypropylmethacrylat-Copolymere,
- anionische Polymere wie beispielsweise Polyacrylsäuren, vernetzte Polyacrylsäuren, Vinylacetat/Crotonsäure-Copolymere, Vinylpyrrolidon/Vinylacrylat-Copolymere, Vinylacetat/Butylmaleat/Isobornylacrylat-Copolymere, Methylvinylether/Maleinsäureanhydrid-Copolymere und Acrylsäure/Ethylacrylat/N-tert.Butylacrylamid-Terpolymere,
- Verdickungsmittel wie Agar-Agar, Guar-Gum, Alginate, Xanthan-Gum, Gummi arabicum, Karaya-Gummi, Johannisbrotkernmehl, Leinsamengummen, Dextrane, Cellulose-Derivate, z. B. Methylcellulose, Hydroxyalkylcellulose und Carboxymethylcellulose, Stärke-Fraktionen und Derivate wie Amylose, Amylopektin und Dextrine, Tone wie z. B. Bentonit oder vollsynthetische Hydrokolloide wie z.B. Polyvinylalkohol,
- Strukturanten wie Glucose und Maleinsäure,
- haarkonditionierende Verbindungen wie Phospholipide, beispielsweise Sojalecithin, Ei-Lecithin und Kephaline,
- Proteinhydrolysate, insbesondere Elastin-, Kollagen-, Keratin-, Milcheiweiß-, Sojaprotein- und Weizenproteinhydrolysate, deren Kondensationsprodukte mit Fettsäuren sowie quaternisierte Proteinhydrolysate,
- Parfümöle, Dimethylisosorbid und Cyclodextrine,
- Lösungsvermittler, wie Ethanol, Isopropanol, Ethylenglykol, Propylenglykol, Glycerin und Diethylenglykol,
- lichtstabile Farbstoffe,
- Antischuppenwirkstoffe wie Zink Omadine,
- weitere Substanzen zur Einstellung des pH-Wertes,
- Wirkstoffe wie Panthenol, Allantoin, Pyrrolidoncarbonsäuren und deren Salze sowie Pflanzenextrakte,
- weitere Lichtschutzmittel,
- Konsistenzgeber wie Zuckerester, Polyolester oder Polyolalkylether,
- Fette und Wachse, wie Walrat, Bienenwachs, Montanwachs und Fettalkohole,
- Fettsäurealkanolamide,
- Komplexbildner wie EDTA, NTA und Phosphonsäuren,
- Quell- und Penetrationsstoffe wie Glycerin, Propylenglykolmonoethylether, Carbonate, Hydrogencarbonate, Guanidine, Harnstoffe sowie primäre, sekundäre und tertiäre Phosphate,
- Antioxidantien,
- direktziehende Farbstoffe,
- sogenannte Kuppler- und Entwicklerkomponenten als Oxidationsfarbstoffvorprodukte,
- Reduktionsmittel wie z.B. Thioglykolsäure und deren Derivate, Thiomilchsäure, Cysteamin, Thioäpfelsäure und α-Mercaptoethansulfonsäure,
- Oxidationsmittel wie Wasserstoffperoxid, Kaliumbromat und Natriumbromat.

Die erfindungsgemäßen Zubereitungen unterliegen hinsichtlich der Art des Mittels keinerlei weiteren Beschränkungen. Bevorzugt sind aber kosmetische Mittel, insbesondere Haarbehandlungsmittel. Innerhalb der Gruppe der Haarbehandlungsmittel sind die erfinderischen Zubereitungen insbesondere geeignet für Haarpflegeprodukte wie Haarkuren, Haarbalsame etc., die ohne weiteres Spülen auf dem Haar verbleiben. Gegenstand der Erfindung ist daher ebenfalls ein Verfahren zur Behandlung von Haaren, bei dem eine erfindungsgemäße Zubereitung auf das Haar aufgebracht wird und dort verbleibt.

Es können aber auch Produkte formuliert werden, die nach einer gewissen Einwirkzeit (üblicherweise ca. 30 Sekunden bis ca. 30 Minuten) wieder aus dem Haar ausgespült werden. Gegenstand der Erfindung ist daher auch ein Verfahren zur Behandlung von Haaren, bei dem eine erfindungsgemäße Zubereitung auf das Haar aufgebracht und nach einer Einwirkzeit wieder ausgespült wird.

Die folgenden Beispiele sollen die Erfindung näher erläutern.

### Beispiele

### Formulierungsbeispiele:

Alle Angaben sind in Gewichts-Teilen.

### 1. Haarkur:

| Ölphase: | |
|---|---|
| Dow Corning^{R}345¹ | 13,0 |
| Finsolv^{R}TN² | 5,0 |
| Uvinul^{R} M 40³ | 2,0 |
| D&C Violet No. 2 (C.I. 60725) | 0,0005 |

| Wasserphase: | |
|---|---|
| Wasser | 77,05 |
| Hostacerin^{R}DGL⁴ | 0,35 |
| Luviquat^{R}FC 370⁵ | 2,0 |
| p-Hydroxybenzoesäure- | |
| methylester | 0,1 |
| Uvinul^{R} MS 40⁶ | 0,5 |
| D&C Yellow No. 6 (C.I. 15985) | 0,0005 |

| | |
|---|---|
| ¹ Decamethylpentasiloxan (CTFA-Bezeichnung: Cyclomethicone) (DOW CORNING) | |
| ² Benzoesäure-C12-15-alkylester (CTFA-Bezeichnung: C12-C15 Alkyl Benzoate) (FINETEX) | |
| ³ 2-Hydroxy-4-methoxy-benzophenon (CTFA-Bezeichnung: Benzophenone-3) (BASF) | |
| ⁴ Fettsäurepolyglycerinester-Oxethylat (CTFA-Bezeichnung: PEG-10 polyglyceryl-2-laurate) (HOECHST) | |
| ⁵ Vinylimidazoliummethochlorid-Vinylpyrrolidon-(30:70)-Copolymer (40 % Aktivsubstanz in Wasser; CTFA-Bezeichnung: Polyquaternium-16) (BASF) | |
| ⁶ 2-Hydroxy-4-methoxy-benzophenon-5-sulfonsäure (CTFA-Bezeichnung: Benzophenone-4) (BASF) | |

### 2. Haarkur:

| Ölphase: | |
|---|---|
| Paraffinöl | 18,5 |
| Escalol^{R}507⁷ | 1,0 |
| Vitamin E-acetat | 0,5 |

| Wasserphase: | |
|---|---|
| Wasser | 78,0 |
| Pathequat^{R} ⁸ | 0,5 |
| Uvinul^{Rp} 25⁹ | 0,5 |
| Luviquat^{R}FC 905¹⁰ | 1,0 |

| | |
|---|---|
| ⁷ N,N-Dimethyl-4-aminobenzoesäure-2-ethylhexylester (CTFA-Bezeichnung: Octyl Dimethyl PABA) (VAN DYK) | |
| ⁸ kationisches Panthenolderivat (45 % Aktivsubstanz; CTFA-Bezeichnung: Panthenyl Hydroxypropylsteardimoniumchlorid) (TRI-K Industries) | |
| ⁹ 1,4-Aminobenzoesäureethylester + 25 EO (CTFA-Bezeichnung: PEG-25 PABA) (BASF) | |
| ¹⁰ Vinylimidazoliummethochlorid-Vinylpyrrolidon-(95:5)-Copolymer (40 % Aktivsubstanz in Wasser; CTFA-Bezeichnung: Polyquaternium-16) (BASF) | |

## Patentansprüche

1. In Form eines 2-Phasen-Systems vorliegende, durch mechanische Einwirkung kurzzeitig mischbare Zubereitung zur Behandlung keratinischer Gebilde, insbesondere menschlicher Haare und menschlicher Haut, dadurch gekennzeichnet, daß sie mindestens einen gelösten, gegen Tageslicht instabilen Inhaltsstoff und, in der gleichen Phase gelöst, einen UV-Filter, enthält.

2. Zubereitung nach Anspruch 1, dadurch gekennzeichnet, daß eine Phase Wasser oder eine Emulsion ist, die bis zu 5 Gew.-% Öl, bezogen auf die Komponenten Wasser und Öl, enthält.

3. Zubereitung nach Anspruch 1 oder 2, dadurch gekennzeichnet, daß die Öl-Phase bis zu 20 Gew.-% Wasser, bezogen auf die Komponenten Öl und Wasser, enthält.

4. Zubereitung nach einem der Ansprüche 1 bis 3, dadurch gekennzeichnet, daß die beiden Phasen in einem Massen-Verhältnis von 1:1 bis 5:1 vorliegen.

5. Zubereitung nach einem der Ansprüche 1 bis 4, dadurch gekennzeichnet, daß es sich bei dem gegen Tageslicht instabilen Inhaltsstoff um einen Farbstoff zum Anfärben der Zubereitung handelt.

6. Zubereitung nach einem der Ansprüche 1 bis 5, dadurch gekennzeichnet, daß es sich bei dem gegen Tageslicht instabilen Inhaltsstoff um ein Vitamin handelt.

7. Zubereitung nach einem der Ansprüche 1 bis 6, dadurch gekennzeichnet, daß der UV-Filter ein Derivat des Benzophenons oder ein Derivat der p-Aminobenzoesäure ist.

8. Verfahren zur Behandlung von Haaren, dadurch gekennzeichnet, daß eine Zubereitung nach einem der Ansprüche 1 bis 7 auf das Haar aufgebracht wird und dort verbleibt.

9. Verfahren zur Behandlung von Haaren, dadurch gekennzeichnet, daß eine Zubereitung nach einem der Ansprüche 1 bis 7 auf das Haar aufgebracht wird und nach einer Einwirkzeit wieder ausgespült wird.

## Claims

1. A composition in the form of a two-phase system briefly miscible by mechanical action for treating keratin structures, more especially human hair and human skin, characterized in that it contains at least one dissolved ingredient unstable in daylight and a UV filter dissolved in the same phase.

2. A composition as claimed in claim 1, characterized in that one phase is water or an emulsion containing up to 5% by weight of oil, based on the components water and oil.

3. A composition as claimed in claim 1 or 2, characterized in that the oil phase contains up to 20% by weight of water, based on the components oil and water.

4. A composition as claimed in any of claims 1 to 3, characterized in that the two phases are present in a ratio by weight of 1:1 to 5:1.

5. A composition as claimed in any of claims 1 to 4, characterized in that the ingredient unstable in daylight is a dye for colouring the composition.

6. A composition as claimed in any of claims 1 to 5, characterized in that the ingredient unstable in daylight in a vitamin.

7. A composition as claimed in any of claims 1 to 6, characterized in that the UV filter is a derivative of benzophenone or a derivative of p-aminobenzoic acid.

8. A hair treatment process, characterized in that the composition claimed in any of claims 1 to 7 is applied to the hair and left thereon.

9. A hair treatment process, characterized in that the composition claimed in any of claims 1 to 7 is applied to the hair and, after a contact time, is rinsed out again.

## Revendications

1. Composition présente sous forme d'un système à deux phases, mélangeable en peu de temps par action mécanique, pour le traitement des édifices kératinisés, en particulier les cheveux humains et la peau humaine, caractérisée en ce qu'elle contient au moins un constituant dissous, instable vis-à-vis de la lumière du jour et, dissous dans la même phase, un filtre U.V.

2. Composition suivant la revendication 1, caractérisée en ce qu'une phase est de l'eau ou une émulsion, qui contient jusqu'à 5% en poids d'huile, sur base des composants eau et huile.

3. Composition suivant la revendication 1 ou 2, caractérisée en ce que la phase huileuse contient jusqu'à 20% en poids d'eau, sur base des composants huile et eau.

4. Composition suivant l'une quelconque des revendications 1 à 3, caractérisée en ce que les deux phases sont présentes dans un rapport massique allant de 1:1 à 5:1.

5. Composition suivant l'une quelconque des revendications 1 à 4, caractérisée en ce que le constituant instable vis-à-vis de la lumière du jour consiste en un colorant pour teindre la composition.

6. Composition suivant l'une quelconque des revendications 1 à 5, caractérisée en ce que le constituant instable vis-à-vis de la lumière du jour consiste en une vitamine.

7. Composition suivant l'une quelconque des revendications 1 à 6, caractérisée en ce que le filtre U.V. est un dérivé de benzophénone ou un dérivé d'acide p-aminobenzoïque.

8. Procédé de traitement des cheveux, caractérisé en ce qu'une composition suivant l'une quelconque des revendications 1 à 7 est appliquée sur les cheveux et y reste.

9. Procédé de traitement des cheveux, caractérisé en ce qu'une composition suivant l'une quelconque des revendications 1 à 7 est appliquée sur les cheveux et est éliminée par rinçage après une période d'action.
